# EUROPÄISCHE PATENTSCHRIFT

(11) **EP 1 096 923 B1**
(45) Veröffentlichungstag und Bekanntmachung des Hinweises auf die Patenterteilung: **07.01.2004**
(21) Anmeldenummer: 99932796.8
(22) Anmeldetag: 02.07.1999
(51) Int. Cl.: A61K 9/70

(54) **BLISTER ENTHALTEND EIN TRANSDERMALES THERAPEUTISCHES SYSTEM UND EINE EINZELDOSIERTE DARREICHUNGSFORM**
BLISTER CONTAINING A TRANSDERMAL THERAPEUTIC SYSTEM AND A SINGLE DOSE FORM OF ADMINISTRATION
PLAQUETTE ALVEOLAIRE CONTENANT UN SYSTEME THERAPEUTIQUE TRANSDERMIQUE ET UNE FORME GALENIQUE INDIVIDUELLE

(30) Priorität: 11.07.1998 DE 19831263
(43) Veröffentlichungstag der Anmeldung: 09.05.2001
(73) Patentinhaber: LTS Lohmann Therapie-Systeme AG, 56626 Andernach (DE)
(72) Erfinder: SCHÄFER, Wolfgang, D-53757 St. Augustin (DE); FRANKE, Hanshermann, D-56566 Neuwied (DE); ASMUSSEN, Bodo, D-56170 Bendorf-Sayn (DE)
(74) Vertreter: Flaccus, Rolf-Dieter, Dr.
(86) Internationale Anmeldenummer: PCT/EP1999/004614
(87) Internationale Veröffentlichungsnummer: WO 2000/002538

(56) Entgegenhaltungen:
- EP-A- 0 307 352
- WO-A-91/09731
- WO-A-99/01132

## Beschreibung

Die Erfindung betrifft eine Darreichungform für medizinische Wirkstoffe in Blisterform, die eine simultane Verabreichung eines transdermalen therapeutischen Systems (TTS) und mindestens eines einzeldosierten Wirkstoffs ermöglicht.

In der Hormon-Replacement-Therapie ist es üblich, die Gabe des Basis-Hormons zum Vermeiden von Nebenwirkungen mit der Gabe eines weiteren Hormons zu kombinieren. Dies trifft z.B. für die Oestradiol-Applikation zu, die mit Gestagengabe erfolgt. Da eine langandauernde, kontinuierliche Gabe des Basishormons angestrebt wird, hat sich die Applika tionsform des transdermalen therapeutischen Systems (TTS) bewährt, bei dem der Wirkstoff in kontrollierter Weise über eine definierte Kontaktfläche eines Wirkstoffreservoirs an die Haut abgegeben wird. Vorzugsweise ist das System haftklebend. Ist die diskontinuierliche Begleitmedikation mittels eines weiteren Hormons erwünscht, so erfolgt dies in der Regel in Tablettenform. Die Tabletten sind meist separat bevorratet und können nur schwer dem zeitlichen Ablauf der Applikation entsprechend gehandhabt werden. Damit ist diese Art der Darreichungsformen der Compliance der Patienten äußerst abträglich.

EP 0 307 352 A1 offenbart eine Verpackung für Arzneimittel, die zwei Plätze enthält, wobei eine der beiden Plätze zur Aufnahme von Einzeldosis-Darreichungsformen bestimmt ist und als Blister- (Durchdrück)-Packung gestaltet sein kann, und der andere Platz für die Aufnahme von transdermalen Pflastern bestimmt ist. Dieser Platz ist vorzugsweise als verschließbare Tasche ausgebildet.

Es war daher Aufgabe der Erfindung, aus Compliance-Gründen für die simultane Verabreichung von medizinischen Wirkstoffen über die transdermale und nicht-transdermale Route eine geeignete verpackungstechnische Maßnahme zu schaffen. Damit sollte sichergestellt worden, daß der Patient durch entsprechende Verpackungsgestaltung eine Hilfestellung bekommt, die zeitlich definierte Einzeldosis-Einnahme nach Möglichkeit zu realisieren.

Die Lösung der Aufgabe wurde nun darin gefunden, daß eine Blisterform neben dem TTS mindestens eine einzeldosierte

Wirkstoffform enthält. Das TTS ist in einem Blister einzeln verpackt, kann aber auch anderweitig auf der Darreichungsform fixiert sein, wie beispielsweise mittels der haftklebenden Kontaktfläche. Auch für die einzeldosierte Wirkstoffform, die vorzugsweise eine Tablette darstellt, ist die Unterbringung in einem Blister die Methode der Wahl, wobei natürlich auch andere Befestigungsmechanismen denkbar sind.

Zur längerdauernden Applikation hat sich das Aneinanderfügen mehrerer Blisterformen als vorteilhaft erwiesen. Dies bedeutet für den Patienten, daß sein Applikationsschema über Wochen exakt in eindeutiger Weise festgelegt ist. In der Serie von Blisterformen können durchaus auch solche enthalten sein, die keine einzeldosierte Wirkstoffform aufweisen, wenn es therapeutische Gründe gebieten.

Die einzeldosierte Wirkstoffform ist in der Regel für eine nicht-transdermale Applikation ausgelegt. Andernfalls muß dafür Sorge getragen werden, daß eine bolusartige Freisetzung gewährleistet ist. Neben oder anstelle von systemischen Wirkstoffen kann die einzeldosierte Wirkstoffform auch Zubereitungen zur topischen Vor- und/oder Nachbehandlung der TTS-Applikationsfläche enthalten. Diese Zubereitungen können beispielsweise die Hautdurchlässigkeit für den Wirkstoff aus dem TTS verbessern oder schädliche Hautveränderungen nach der Applikation des TTS verhindern oder gar rückgängig machen. Nicht zuletzt kann die einzeldosierte Wirkstoffform auch einen oder mehrere Wirkstoffe des TTS enthalten, um die Lagtime des TTS zu überbrücken oder einen zirkadian erforderlichen Plasmazusatzpeak zu erzeugen.

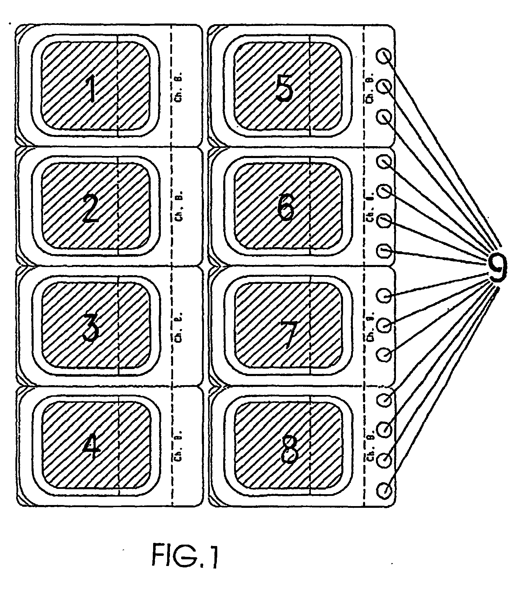

Das nachfolgende Beispiel, illustriert durch FIG.1, faßt die Compliance-sicheren Aspekte der neuen Darreichungsform mit Hilfe einer aus acht TTS 1 bis 8 zusammengefügten Einheit zusammen, die für einen Behandlungszeitraum von 28 Tagen vorgesehen ist. Mit 9 sind die einzeldosierten Wirkstoffformen gekennzeichnet, wobei 1-4 keine solchen enthalten.
Die folgende Tabelle spiegelt den Applikationsverlauf für eine Oestrogen-Gestagen-Behandlung wieder:

| | Nr. der Blisterform | Tragdauer der Blisterform |
|---|---|---|
| Oes-Behandlung | Blister Nr. 1 | 1.,2.,3. Tag |
| ohne | Blister Nr. 2 | 4.,5.,6.,7. Tag |
| Gestagen-Gabe | Blister Nr. 3 | 8.,9.,10. Tag |
| | Blister Nr. 4 | 11.,12.,13.,14. Tag |
| Oes-Behandlung in Kombination mit Gestagen-Gabe in Tablettenform | Blister Nr. 5 | 15.,16.,17. Tag |
| | Blister Nr. 6 | 18.,19.,20.,21. Tag |
| | Blister Nr. 7 | 22.,23.,24. Tag |
| | Blister Nr. 8 | 25.,26.,27.,28. Tag |

Nach einer Gestagen-freien Behandlungsdauer von 14 Tagen erfolgt in den letzten beiden Wochen für jeden Tag der TTS-Applikation (alternierend drei und vier Tage) die Bereitstellung einer Gestagen-Gabe.

Wie das Beispiel erläutert, stellt die Erfindung ein sicheres Mittel zur Verfügung, um die Compliance der Patienten bei der TTS-Applikation mit einer zusätzlichen einzeldosierten Wirkstoffform nachhaltig zu verbessern.

Zur Erhöhung der Compliance können mehrere Blisterformen aneinandergefügt oder die einzelnen Blister in der gewünschten Reihenfolge der Nutzung gekennzeichnet sein.

## Patentansprüche

1. Blister-Verpackung für Arzneimittel, welche transdermale therapeutische Systeme und einzeldosierte Wirkstoffformen enthält, **dadurch gekennzeichnet, daß** sie mindestens eine Blister-Form umfaßt, welche nebeneinander ein einzelnes transdermales therapeutisches System und zugleich mindestens eine einzeldosierte Wirkstofform enthält.

2. Blister-Verpackung nach Anspruch 1, **dadurch gekennzeichnet, daß** zur Erhöhung der Compliance mehrere Blisterformen aneinandergefügt sind.

3. Blister-Verpackung nach Anspruch 1, **dadurch gekennzeichnet, daß** zur Erhöhung der Compliance die einzelnen Blister in der gewünschten Reihenfolge der Nutzung **gekennzeichnet** sind.

4. Blister-Verpackung nach Anspruch 2 oder 3, **dadurch gekennzeichnet, daß** auch Blisterformen ohne zusätzliche einzeldosierte Wirkstoffformen enthalten sein können.

5. Blister-Verpackung nach einem oder mehreren der Ansprüche 1 bis 4, **dadurch gekennzeichnet, daß** die einzeldosierte Wirkstoffform zu einer nicht-transdermalen Applikation des Wirkstoffs bestimmt ist.

6. Blister-Verpackung nach einem oder mehreren der Ansprüche 1 bis 5, **dadurch gekennzeichnet, daß** die einzeldosierte Wirkstoffform Zubereitungen zur topischen Vorund/oder Nachbehandlung der TTS-Applikation enthält.

7. Blister-Verpackung nach einem oder mehreren der Ansprüche 1 bis 6, **dadurch gekennzeichnet, daß** die einzeldosierten Wirkstoffformen einen oder mehrere der über das TTS applizierten Wirkstoffe enthält.

## Claims

1. Blister pack for pharmaceuticals which contains transdermal therapeutic systems and single-dose active ingredient forms, **characterized in that** it comprises at least one blister form containing, alongside each other, a single transdermal therapeutic system and at the same time at least one single-dose active ingredient form.

2. Blister pack according to Claim 1, **characterized in that** several blister forms are joined together to increase compliance.

3. Blister pack according to Claim 1, **characterized in that** the individual blisters are labelled in the required sequence of use to increase compliance.

4. Blister pack according to Claim 2 or 3, **characterized in that** blister forms without additional single-dose active ingredient forms may also be present.

5. Blister pack according to one or more of Claims 1 to 4, **characterized in that** the single-dose active ingredient form is intended for non-transdermal administration of the active ingredient.

6. Blister pack according to one or more of Claims 1 to 5, **characterized in that** the single-dose active ingredient form contains preparations for topical treatment before and/or after the TTS administration.

7. Blister pack according to one or more of Claims 1 to 6, **characterized in that** the single-dose active ingredient forms contain one or more of the active ingredients administered via the TTS.

## Revendications

1. Conditionnement de plaquette alvéolaire pour médicaments, lequel contient des systèmes thérapeutiques transdermiques et des formes de substance active individuelles, **caractérisé en ce qu'**il comprend au moins une forme de plaquette alvéolaire, laquelle contient l'un à côté de l'autre un système thérapeutique transdermique individuel et en même temps au moins une forme de substance active individuelle.

2. Conditionnement de plaquette alvéolaire selon la revendication 1, **caractérisé en ce que** plusieurs formes de plaquettes alvéolaires sont placées les unes à côté des autres pour augmenter la conformité.

3. Conditionnement de plaquette alvéolaire selon la revendication 1, **caractérisé en ce que** les plaquettes alvéolaires individuelles sont marquées dans l'ordre souhaité d'utilisation pour augmenter la conformité.

4. Conditionnement de plaquette alvéolaire selon la revendication 2 ou 3, **caractérisé en ce que** des formes de plaquette alvéolaires sans formes supplémentaires de substance active individuelles peuvent être également contenues.

5. Conditionnement de plaquette alvéolaire selon une ou plusieurs des revendications 1 à 4, **caractérisé en ce que** la forme individuelle de substance active est destinée à une application non transdermique de la substance active.

6. Conditionnement de plaquette alvéolaire selon une ou plusieurs des revendications 1 à 5, **caractérisé en ce que** la forme individuelle de substance active contient des préparations pour le traitement topique préalable et/ou ultérieur de l'application TTS.

7. Conditionnement de plaquette alvéolaire selon une ou plusieurs des revendications 1 à 6, **caractérisé en ce que** les formes individuelles de substance active contiennent une ou plusieurs des substances actives appliquées au moyen du TTS.
